# EUROPEAN PATENT APPLICATION

(11) **EP 2 275 035 A1**
(43) Date of publication of application: **19.01.2011**
(21) Application number: 09746402.8
(22) Date of filing: 09.01.2009
(51) Int. Cl.: A61B 5/151, A61B 5/1486, A61B 5/157, G01N 27/02

(54) **BODY-LIQUID SAMPLING CIRCUIT BOARD, ITS MANUFACTURING METHOD AND ITS USING METHOD, AND BIOSENSOR HAVING THE BODY-LIQUID SAMPLING CIRCUIT BOARD**

(30) Priority: 16.05.2008 JP 2008129827
(71) Applicant: Nitto Denko Corporation, Ibaraki-shi Osaka 567-8680 (JP)
(72) Inventor: KANETO, Masayuki, Ibaraki-shi Osaka 567-8680 (JP); OHSAWA, Tetsuya, Ibaraki-shi Osaka 567-8680 (JP); ISHIGAKI, Saori, Ibaraki-shi Osaka 567-8680 (JP); NAITO, Toshiki, Ibaraki-shi Osaka 567-8680 (JP)
(74) Representative: HOFFMANN EITLE
(86) International application number: PCT/JP2009/050243
(87) International publication number: WO 2009/139195

(57) **Abstract**

A circuit board for body fluid collection includes a belt-like supporting substrate, and a plurality of measurement units defined in the supporting substrate along the longitudinal direction of the supporting substrate. The measurement units each include a puncture needle formed from the supporting substrate along the longitudinal direction of the supporting substrate by forming an opening in the supporting substrate, an insulating base layer formed on the supporting substrate, and a conductive layer formed on the insulating base layer and including an electrode for making contact with a body fluid collected by puncturing with the puncture needle.

## Description

### TECHNICAL FIELD

The present invention relates to a circuit board for body fluid collection, a method for producing the circuit board for body fluid collection, a method for using the circuit board for body fluid collection, and a biosensor including the circuit board for body fluid collection.

### BACKGROUND ART

Diabetes mellitus includes insulin-dependent (type I) diabetes and non-insulin-dependent (type II) diabetes. The former type of diabetes necessitates regular administration of insulin. Therefore, for a patient with the former type of diabetes, there has been employed a treatment method in which a patient collects his or her blood, measures his or her blood sugar level, and administers to himself or herself insulin at a dosage in accordance with the blood sugar level.
There has been known, for mainly such patients, a blood-sugar-level measuring device which allows a patient to personally collect blood on his/her own and to measure a blood sugar level.

For example, there has been proposed a fluid collecting device including a reaction zone which is provided at the center of a main body and into which electrodes are inserted; a puncture needle outwardly protruding from the center of the main body; and a capillary channel providing communication between the electrodes and the puncture needle (ref: for example, Patent Document 1 shown below).
Patent Document 1
Japanese Unexamined Patent Publication No. 2004-493

### DISCLOSURE OF THE INVENTION

### PROBLEM TO BE SOLVED BY THE INVENTION

In the above-described fluid collecting device of Patent Document 1, the puncture needle and the reaction zone are formed integrally with the main body, and therefore the measurement preparation is easy. However, with this fluid collecting device, the electrode which is a member separate from the reaction zone has to be inserted into the reaction zone to perform a measurement on the blood component. Therefore, there is a disadvantage in that blood detection accuracy becomes unstable and accurate measurement cannot be performed.
Moreover, in type I diabetes, depending on symptoms, the patient has to measure the blood-sugar level several times per day, to be specific, before every meal or after every meal.

However, with the above-described fluid collecting device of Patent Document 1, only one puncture needle is provided in one device, and therefore in order to avoid repetitive use of the puncture needle, the measurement can be performed only once.
Therefore, when the measurement is performed several times as described above with the above-described fluid collecting device, it is necessary that the used fluid collecting device is disposed and a new fluid collecting device is prepared afterwards. Thus, with such a fluid collecting device, the measurement preparation as described above is complicated, and an increase in running costs is inevitable.

An object of the present invention is to provide a circuit board for body fluid collection that is capable of accurate measurement on a body fluid component with a simple structure, and even capable of easy measurement a plurality of times with one circuit board for body fluid collection; a method for producing the circuit board for body fluid collection; a method for using the circuit board for body fluid collection; and a biosensor including the circuit board for body fluid collection.

### MEANS FOR SOLVING THE PROBLEM

To achieve the above object, a circuit board for body fluid collection of the present invention includes: a belt-like supporting substrate, and a plurality of measurement units defined in the supporting substrate along the longitudinal direction of the supporting substrate, wherein the plurality of measurement units each include a puncture needle formed from the supporting substrate along the longitudinal direction of the supporting substrate by forming an opening in the supporting substrate, an insulating base layer formed on the supporting substrate, and a conductive layer formed on the insulating base layer and including an electrode for making contact with a body fluid collected by puncturing with the puncture needle.

The circuit board for body fluid collection includes the measurement unit including the puncture needle and the electrode. Thus, by causing a body fluid to flow out by puncturing with the puncture needle, the body fluid that was caused to flow out is easily brought into contact with the electrode in the measurement unit. As a result, with the circuit board for body fluid collection, a measurement on a component in body fluid can be performed easily with a simple structure.
Moreover, with the circuit board for body fluid collection, a measurement on a component in body fluid can be performed a plurality of times using the plurality of measurement units provided in one circuit board for body fluid collection.

Furthermore, in the circuit board for body fluid collection, the plurality of measurement units are disposed along the circumferential direction when the circuit board for body fluid collection is wound, and therefore after using one measurement unit, the used measurement unit can be changed to an unused measurement unit that is adjacent to and at an upstream of the used measurement unit in a rotational direction by rotating the circuit board for body fluid collection in the circumferential direction. Therefore, the measurement unit can be changed easily.

In the circuit board for body fluid collection of the present invention, it is preferable that, when the circuit board for body fluid collection is wound, the puncture needle protrudes so as to tilt toward an outer side in the radial direction from the circumferential direction.
With the circuit board for body fluid collection, the puncture needle is disposed at an outer side in the radial direction of the circuit board for body fluid collection that is wound, and therefore reliable puncturing and measurement by the measurement unit can be achieved.

Furthermore, a biosensor of the present invention includes the above-described circuit board for body fluid collection; a housing in which an exposure slot for exposing the puncture needle is formed; a feed unit in which the circuit board for body fluid collection is wound, and that feeds the circuit board for body fluid collection; a take-up unit that takes up the circuit board for body fluid collection that is fed from the feed unit; and a guide unit that is provided to extend from the feed unit to the take-up unit, and suppresses the puncture needle of the circuit board for body fluid collection not to protrude toward an outer side in the radial direction.

In this biosensor, as the take-up unit rotates and takes up the circuit board for body fluid collection, the measurement unit moves from the feed unit to the take-up unit. At this time, the guide unit that is provided to extend from the feed unit to the take-up unit suppresses protrusion of the puncture needle.
Then, when the measurement unit reaches the exposure slot, the puncture needle is brought away from the guide unit, protrudes toward an outer side in the radial direction, and is exposed from the exposure slot. Thus, at an outer side in the radial direction of the circuit board for body fluid collection that is wound, reliable puncturing and measurement by the measurement unit can be achieved.

In the biosensor of the present invention, it is preferable that the puncture needle is disposed to face toward an upstream-side in the feed direction of the circuit board for body fluid collection.
That is, in the above-described biosensor, when the puncture needle is disposed to face toward a downstream-side in the feed direction of the circuit board for body fluid collection, the puncture needle is pressed toward an upstream-side in the feed direction due to friction against the guide unit, and the direction of the puncture needle and the direction of the pressing force of pressing by the guide unit on the puncture needle oppose each other. Therefore, the puncture needle may be damaged due to the friction against the guide unit. Furthermore, in the take-up unit, the puncture needle at an inner side in the radial direction may damage the circuit board for body fluid collection at an outer side in the radial direction.

However, when the puncture needle is disposed to face toward an upstream-side in the feed direction of the circuit board for body fluid collection, both of the direction of the puncture needle and the direction of the pressing force of pressing by the guide unit on the puncture needle are facing toward an upstream-side in the feed direction. Therefore, the puncture needle can fend off the pressing force from the guide unit by getting away along the guide unit toward an inner side in the radial direction of the circuit board for body fluid collection that is wound. Thus, the circuit board for body fluid collection can be transported smoothly without damage to the circuit board for body fluid collection or the puncture needle.

A method for producing a circuit board for body fluid collection of the present invention includes the steps of: preparing a belt-like supporting substrate in which a plurality of measurement units are defined in the longitudinal direction with a space provided therebetween, forming an insulating base layer on the plurality of measurement units, forming a conductive layer including an electrode for making contact with a body fluid on the insulating base layer, and forming a puncture needle from the supporting substrate along the longitudinal direction of the supporting substrate by forming an opening in the supporting substrate.

With the method for producing a circuit board for body fluid collection, the plurality of measurement units can be formed in such a manner that the plurality of measurement units are arranged in line along the longitudinal direction of the belt-like supporting substrate. Therefore, yields of the circuit board for body fluid collection can be improved, and an improvement in production efficiency allows reduction in costs.
A method for using a circuit board for body fluid collection of the present invention includes: winding a circuit board for body fluid collection produced by the above-described method for producing a circuit board for body fluid collection.

With the method for using a circuit board for body fluid collection, by winding the circuit board for body fluid collection, the puncture needle can be protruded so as to tilt toward an outer side in the radial direction from the circumferential direction. Therefore, the puncture needle can be protruded with simple steps. As a result, at an outer side in the radial direction of the circuit board for body fluid collection that is wound, reliable puncturing and measurement by the measurement unit can be achieved.

### EFFECT OF THE INVENTION

With the circuit board for body fluid collection according to the present invention, a measurement on a component in body fluid can be performed a plurality of times with the measurement unit that is provided in a plural number in one circuit board for body fluid collection while an easy measurement on a component in body fluid is achieved with a simple structure. Furthermore, at every measurement in a plurality of measurements, the measurement unit can be easily changed.
Furthermore, with the biosensor according to the present invention, a measurement on a component in body fluid can be easily performed.

Furthermore, with the method for producing a circuit board for body fluid collection of the present invention, yields of the circuit board for body fluid collection can be improved, and an improvement in production efficiency allows reduction in costs.
Furthermore, with the method for using a circuit board for body fluid collection of the present invention, the puncture needle can be protruded toward an outer side in the radial direction of the wound circuit board for body fluid collection with simple steps.

### BRIEF DESCRIPTION OF THE DRAWINGS

FIG. 1 shows a plan view of a circuit board for blood collection as an embodiment of the circuit board for body fluid collection of the present invention.
FIG. 2 shows an enlarged plan view of a measurement unit of the circuit board for blood collection shown in FIG. 1.
FIG. 3 shows a cross-sectional view taken along line A-A in FIG. 2.
FIG. 4 shows production steps for describing an embodiment of a method for producing a circuit board for blood collection of the present invention:
   (a) illustrating a step of preparing a metal substrate,
   (b) illustrating a step of forming an insulating base layer,
   (c) illustrating a step of forming a conductive pattern, and
   (d) illustrating a step of forming an insulating cover layer.
FIG. 5 shows production steps subsequent to FIG. 4 for describing a method for producing a circuit board for blood collection:
   (e) illustrating a step of trimming, and
   (f) illustrating a step of applying a chemical agent.
FIG. 6 illustrates an embodiment of a method for using a circuit board for body fluid collection of the present invention:
   (a) showing a perspective view, and
   (b) showing a schematic side view along the axial direction.
FIG. 7 shows a schematic perspective view of a blood-sugar-level measuring device as an embodiment of a biosensor of the present invention, in which the circuit board for blood collection shown in FIG. 6 is mounted.
FIG. 8 shows a schematic cross-sectional view illustrating inside the blood-sugar level measuring device shown in FIG. 7.
FIG. 9 shows sectional side views for describing a method for using the blood-sugar-level measuring device shown in FIG. 7.
FIG. 10 shows a schematic cross-sectional view of another embodiment of a biosensor of the present invention.

### EMBODIMENT OF THE INVENTION

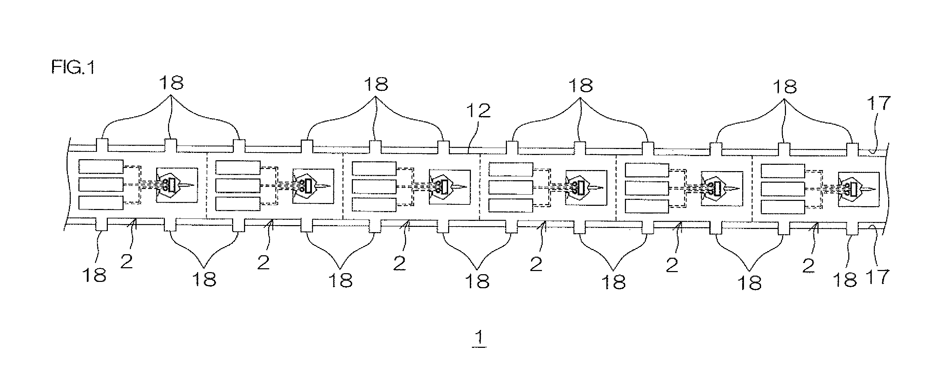

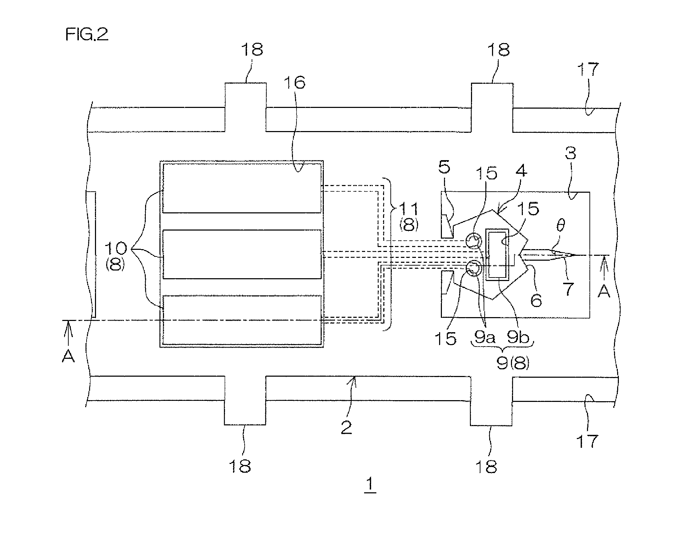

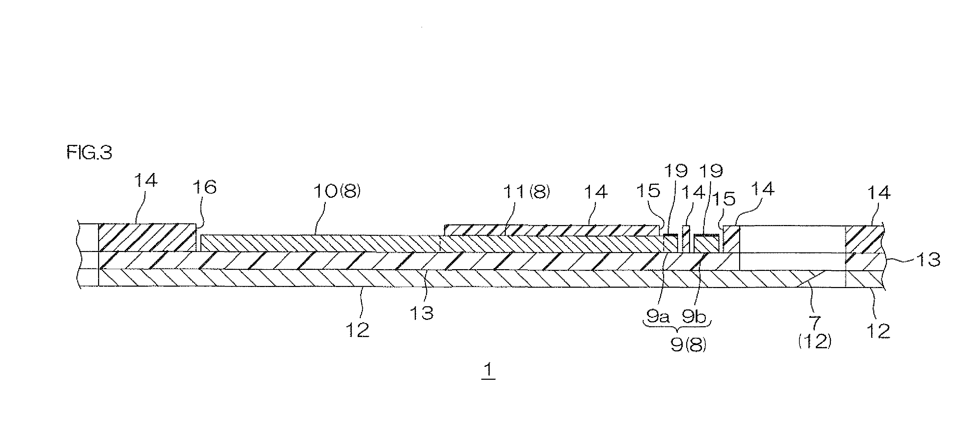

FIG 1. shows a plan view of a circuit board for blood collection as an embodiment of the circuit board for body fluid collection of the present invention; FIG. 2 shows an enlarged plan view of a measurement unit of the circuit board for blood collection shown in FIG. 1; FIG. 3 shows a cross-sectional view taken along line A-A in FIG. 2; and FIGs. 4 and 5 show production steps for describing an embodiment of a method for producing a circuit board for blood collection of the present invention.
A circuit board for blood collection 1 is formed, as shown in FIG. 1, like an elongated belt extending along the longitudinal direction (left-right direction in FIG. 1, including the puncturing direction to be described later. The puncturing direction is a direction toward the distal end of the puncture needle 7 (described later), and in FIG. 1, the puncturing direction corresponds to the direction from the left side to the right side. The same applies to the following.), and although not shown, at the time of its production, a plurality of the circuit boards for blood collection 1 are arranged in parallel along the direction (in the following, referred to as the width direction) perpendicular to the longitudinal direction. The circuit board for blood collection 1 is, as shown in FIG. 1, removably supported by an elongated frame portion 17 via a joint portion 18. The joint portion 18 is extended between the frame portion 17 and the circuit board for blood collection 1.

The circuit board for blood collection 1 includes a metal substrate 12 as a belt-like supporting substrate, and a plurality of measurement units 2 that are defined in the metal substrate 12 along the longitudinal direction of the metal substrate 12.
The size of the circuit board for blood collection 1 is appropriately selected according to the size of a casing 22 (described later) and the number of the measurement units 2, and the length in the longitudinal direction is, for example, 5 to 100 cm, or preferably 15 to 40 cm, although not limited thereto. When the length in the longitudinal direction of the circuit board for blood collection 1 is below the above-described range, the number of the measurement unit 2 may become excessively small. On the other hand, when the length in the longitudinal direction of the circuit board for blood collection 1 exceeds the above-described range, the size of a blood-sugar level measuring device 21 (described later) in which the circuit board for blood collection 1 is mounted becomes large, and may cause a decrease in handleability.

The length in the width direction of the circuit board for blood collection 1 is 2 to 30 mm, or preferably 5 to 15 mm. When the length in the width direction of the circuit board for blood collection 1 is below the above-described range, electrodes 9 (described later) become excessively small, and may cause a decrease in usability. On the other hand, when the length in the width direction of the circuit board for blood collection 1 exceeds the above-described range, the size of the blood-sugar level measuring device 21 (described later) in which the circuit board for blood collection 1 is mounted becomes large, and may cause a decrease in handleability.

The measurement unit 2 is defined like a generally rectangular shape elongated in the longitudinal direction of the circuit board for blood collection 1 when viewed from top, and is disposed to be adjacent to each other in the longitudinal direction of the circuit board for blood collection 1.
The measurement unit 2 includes, as shown in FIG. 2, terminals 10 at an upstream-side end portion in the puncturing direction. At a downstream-side end portion of the measurement unit 2 in the puncturing direction, an opening 3 is formed.
Three terminals 10 are provided to be connected to a CPU 35 (described later) in correspondence with three electrodes 9 (described later). The three terminals 10 are arranged in parallel along the width direction with a space provided therebetween. The terminals 10 are formed like a generally rectangular shape along the longitudinal direction when viewed from top, and the length of a side of the terminal 10 is, for example, 0.5 to 5 mm.

The opening 3 is formed like a generally rectangular shape when viewed from top, and in the opening 3, a connecting portion 5, an electrode portion 4, and a puncture needle 7 are disposed integrally.
The connecting portion 5 is formed like a generally trapezoid shape when viewed from top, being tapered from an upstream side in the puncturing direction toward a downstream-side in the puncturing direction, and is provided so as to protrude from an upstream side end portion of the opening 3 in the measurement unit 2 toward a downstream-side in the puncturing direction.

The electrode portion 4 is formed like a generally pentagon shape when viewed from top, and is formed from the connecting portion 5 toward a downstream-side. The electrode portion 4 includes the electrodes 9 and a stopper portion 6.
The electrodes 9 are provided so as to make contact with blood collected by puncturing with the puncture needle 7, and three electrodes 9 are provided so as to be adjacent to each other in the width direction and in the puncturing direction in the electrode portion 4.
To be specific, two electrodes 9a among the three electrodes 9 are arranged to face each other with a space provided therebetween in the width direction in the electrode portion 4. The two electrodes 9a are formed into a generally circular shape when viewed from top.

The remaining one electrode 9b is disposed to face the two electrodes 9a at a downstream-side in the puncturing direction with a space provided therebetween in the electrode portion 4. The electrode 9b is formed into a generally rectangular shape when viewed from top, and extends over the two electrodes 9a in the width direction.
The three electrodes 9 correspond to a working electrode, a counter electrode, and a reference electrode, respectively. The two electrodes 9a have a diameter of, for example, 100 µm to 5 mm, and the length of a side of the one electrode 9b is, for example, 100 µm to 2.5 mm.

The stopper portion 6 is provided at a downstream-side end portion in the puncturing direction in the electrode portion 4, so as to prevent the puncture needle 7 to deeply pierce the skin excessively. To be specific, the stopper portion 6 is formed, in the electrode portion 4, such that the peak in the puncturing direction at the most downstream-side of the generally regular pentagon shape when viewed from top is dented toward an upstream side in the puncturing direction. That is, the stopper portion 6 is provided, in the electrode portion 4, so as to protrude from both outer sides in the width direction with the puncture needle 7 interposed therebetween.

The puncture needle 7 is provided along the longitudinal direction of the metal substrate 12, so as to collect blood as a body fluid by puncturing. The puncture needle 7 protrudes from a center in the width direction of the electrode portion 4 at a downstream-side end portion in the puncturing direction toward a downstream-side in the puncturing direction. The puncturing direction of the puncture needle 7 in respective measurement units 2 is the same direction (right direction in FIG. 1). The puncture needle 7 is formed into a generally triangle shape (isosceles triangle) when viewed from top, and its distal end (downstream-side end portion in the puncturing direction) is tapered along the puncturing direction to form an acute angle.

Angle θ of the distal end of the puncture needle 7 is, for example, 10 to 30°, or preferably 15 to 25°. When angle θ of the distal end is below 10°, the skin puncturing may not be performed because of insufficient strength. On the other hand, when angle θ exceeds 30°, the puncturing may become difficult. The length of the puncture needle 7 in the puncturing direction is, for example, 0.5 to 10 mm, and the length of the puncture needle 7 in the width direction (the width of an upstream side portion in the puncturing direction) is, for example, 0.1 to 3 mm. When the length in the puncturing direction and the width of the puncture needle 7 are below the above-described range, the blood collection may become difficult, and when the length in the puncturing direction and the width of the puncture needle 7 exceed the above-described range, damage at the punctured portion may increase.

The distal end of the puncture needle 7, and the downstream-side end edge of the stopper portion 6 in the puncturing direction are spaced apart by, for example, 0.3 to 2 mm.
The distal end of the puncture needle 7 is disposed within, for example, 0.2 to 5 mm, or preferably 0.5 to 3 mm from the three electrodes 9 in the puncturing direction.
The measurement unit 2 is provided with three wirings 11 at a middle portion along the longitudinal direction of the measurement unit 2.

The wirings 11 are provided over the upstream side end portion in the puncturing direction and the downstream-side end portion in the puncturing direction of the measurement unit 2, so as to electrically connect respective electrodes 9 and terminals 10 corresponding to the electrodes 9. To be specific, the wirings 11 are provided with a space provided therebetween in the width direction.
The wirings 11 at both sides in the width direction are formed into a generally crank shape, i.e., when viewed from top, the wirings 11 at both sides in the width direction extend from the terminals 10 at both sides in the width direction toward a downstream-side in the puncturing direction; thereafter bend at generally a center of the measurement unit 2 in the longitudinal direction toward an inner side in the width direction; extend to generally a center of the measurement unit 2 in the width direction; and bend toward a downstream-side in the puncturing direction, to be connected to the electrodes 9a passing through a center of the connecting portion 5 in the width direction.

The wiring 11 at the center is formed into a straight line along the puncturing direction when viewed from top, and connects the center terminal 10 and the electrode 9b.
The length of the wirings 11 in the width direction is, for example, 0.01 to 2 mm. The space between the adjacent wirings 11 in the width direction is, at an upstream side in the puncturing direction, 0.05 to 10 mm, and at a downstream-side in the puncturing direction, 0.01 to 1 mm.

The respective electrodes 9, respective terminals 10, and respective wirings 11 that allow connection between them are provided continuously and integrally as a conductive pattern 8.
The circuit board for blood collection 1 includes, as shown in FIG. 3, a metal substrate 12 as a supporting substrate, an insulating base layer 13 laminated on the metal substrate 12, a conductive pattern 8 laminated on the insulating base layer 13, and an insulating cover layer 14 provided on the insulating base layer 13 so as to cover the conductive pattern 8.
The metal substrate 12 is made of, for example, a metal foil, and in the circuit board for blood collection 1, is formed into a belt-like shape corresponding to its outline shape.

Examples of the metal material that forms the metal substrate 12 include nickel, chromium, iron, and stainless steel (SUS304, SUS430, and SUS316L). Preferably, in view of rigidity, stainless steel is used. The thickness of the metal substrate 12 is, for example, 10 to 300 µm, or preferably 20 to 100 µm. When the thickness is below 10 µm, the skin puncturing (described later) may not be performed because of insufficient strength. On the other hand, when the thickness exceeds 300 µm, the puncturing may cause pain and damage the skin excessively.

The insulating base layer 13 is formed so as to cover a portion corresponding to the conductive pattern 8 and the electrode portion 4 of the metal substrate 12, and to expose the puncture needle 7. The insulating base layer 13 is formed into a similar shape having a size slightly larger than the metal substrate 12 in the electrode portion 4, and the peripheral end portion of the insulating base layer 13 extends from the peripheral end portion of the metal substrate 12.
Examples of the insulating material that forms the insulating base layer 13 include synthetic resins such as polyimide resin, polycarbonate resin, polyethylene resin, polyethyleneterephthalate resin, epoxy resin, and fluorocarbon resin. In view of mechanical durability, and chemical resistance, preferably, polyimide resin is used. The thickness of the insulating base layer 13 is, for example, 3 to 50 µm, or preferably 5 to 25 µm. When the thickness is below 3 µm, there may be a case where an insulation defect such as pinholes is caused. On the other hand, when the thickness exceeds 50 µm, cutting and trimming may become difficult.

The conductive pattern 8 is formed on the insulating base layer 13, and is formed as a wired circuit pattern including the above-described three electrodes 9, three terminals 10, and three wirings 11.
Examples of the conductive material that forms the conductive pattern 8 include metal materials such as iron, nickel, chromium, copper, gold, silver, platinum, and alloys thereof. The conductive material is selected appropriately in view of adhesiveness to the insulating base layer 13 and the insulating cover layer 14, and easy workability. Two or more conductive materials may be laminated as well. The thickness of the conductive pattern 8 is, for example, 5 to 50 µm, or preferably 10 to 20 µm.

The insulating cover layer 14 is provided on the insulating base layer 13 so as to cover the wirings 11 and to expose the terminals 10 and electrodes 9. The peripheral end portion of the insulating cover layer 14 is disposed, as shown in FIGs. 2 and 3, so as to coincide with the peripheral end portion of the insulating base layer 13 when viewed from top.
The insulating cover layer 14 is formed with electrode-side openings 15 to expose the electrodes 9, and a terminal-side opening 16 to expose the terminals 10. To be specific, the electrode-side openings 15 are formed, as shown in FIG. 2, to be slightly larger than the electrodes 9 when viewed from top, so as to encircle the electrodes 9. The terminal-side opening 16 is formed into a generally rectangular shape when viewed from top, so as to include all the terminals 10. The insulating cover layer 14 covers the wirings 11. For the insulating material that forms the insulating cover layer 14, the above-described insulating materials of the insulating base layer 13 are used. The thickness of the insulating cover layer 14 is, for example, 2 to 50 µm.

As shown in FIG. 2, the stopper portion 6 is formed from the end portion in the puncturing direction of the insulating base layer 13 and the insulating cover layer 14 extending from the metal substrate 12 in the electrode portion 4. The insulating material that forms the stopper portion 6 is softer than the metal material, and therefore when preventing excessive puncturing with the puncture needle 7, damage to the skin that makes contact with the stopper portion 6 can be effectively prevented.

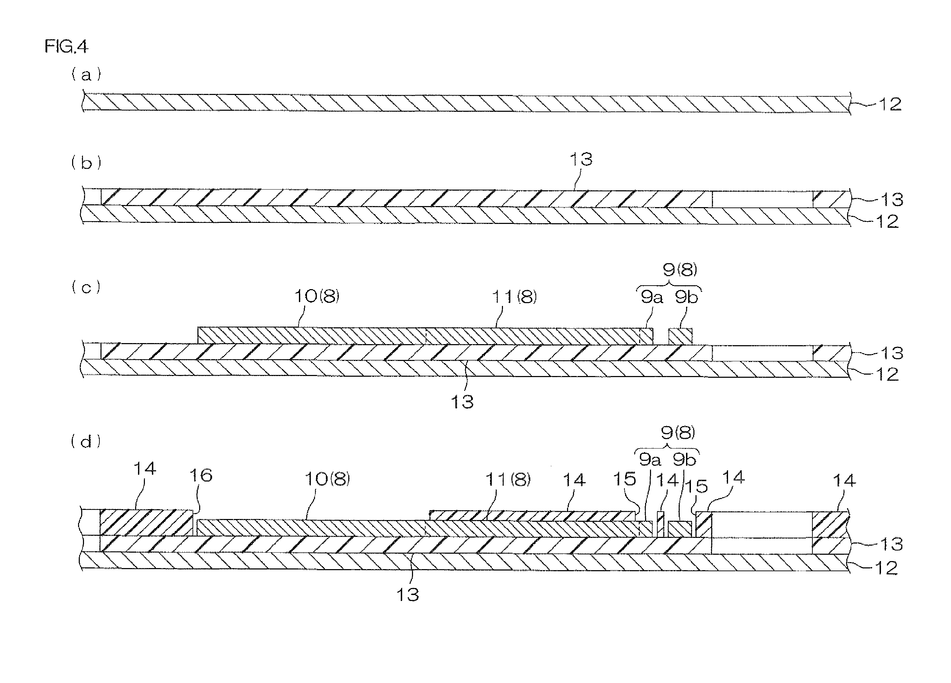

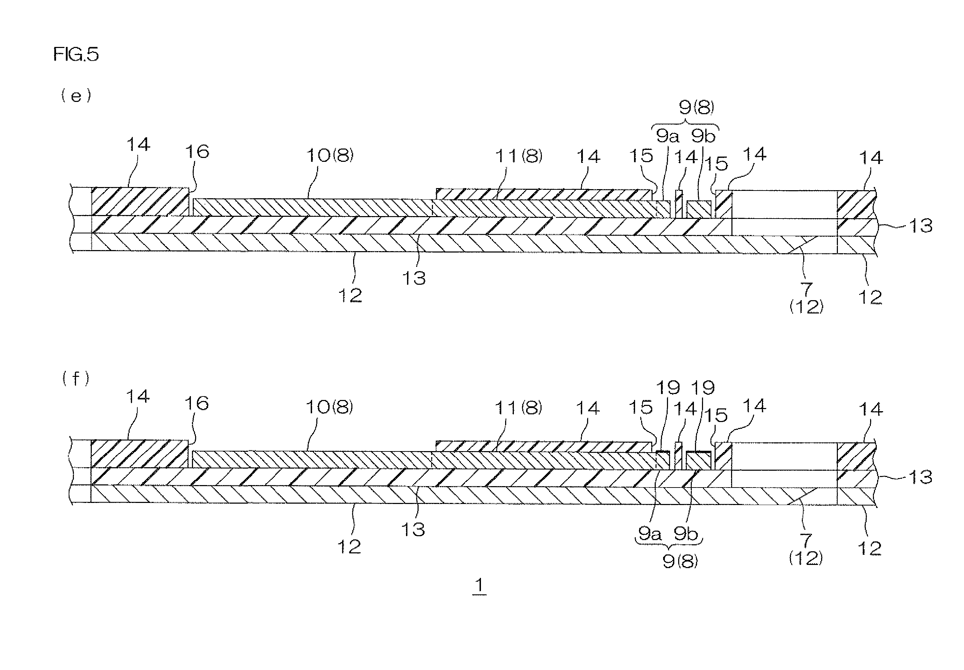

Next, with reference to FIGs. 4 and 5, a method for producing the circuit board for blood collection 1 is described. FIGs. 4 and 5 show cross-sectional views in correspondence with FIG. 3.
In this method, first, as shown in FIG. 4 (a), the belt-like metal substrate 12 in which the plurality of measurement units 2 are defined in the longitudinal direction with a space provided therebetween is prepared. Although not shown, the belt-like metal substrate 12 is defined in a plural number in an elongated sheet metal foil.

Next, in this method, as shown in FIG. 4 (b), the insulating base layer 13 is formed on the measurement unit 2 defined in the metal substrate 12. For the formation of the insulating base layer 13, for example, the following methods are used: a method in which a varnish of a photosensitive synthetic resin is applied on the surface of the metal substrate 12, photoprocessed, and then cured; a method in which a synthetic resin film is laminated onto the surface of the metal substrate 12, an etching resist having the same pattern as that of the insulating base layer 13 is laminated onto the surface of the film, and afterwards, the film exposed from the etching resist is wet-etched; a method in which a synthetic resin film that is punched by a machine in advance is laminated onto the surface of the metal substrate 12; and a method in which a synthetic resin film is laminated onto the surface of the metal substrate 12 first, and then subjected to discharge processing or laser processing. In view of processing accuracy, the method in which a varnish of a photosensitive synthetic resin is applied on the surface of the metal substrate 12, photoprocessed, and then cured is preferably used.

Next, in this method, as shown in FIG. 4 (c), the conductive pattern 8 is formed on the insulating base layer 13 in the above-described pattern. For the formation of the conductive pattern 8, a known patterning method for forming printed wirings is used, such as an additive method and a subtractive method. In view of achieving a minute pattern, preferably, the additive method is used.
In the additive method, for example, a metal thin film is formed on the surface of the insulating base layer 13, by chemical vapor deposition or sputtering, and after a plating resist is formed on the surface of the metal thin film, a plating layer is formed on the surface of the metal thin film exposing from the plating resist by electrolytic plating using the metal thin film as a seed film.

The conductive pattern 8 can also be formed only of the metal thin film by chemical vapor deposition or sputtering.
Upon formation of the conductive pattern 8, a different type of metal plating layer may also be formed on the surface of the electrodes 9 and the surface of the terminals 10 by further electrolytic plating or electroless plating, although not shown in the drawings. The thickness of the metal plating layer is preferably 0.05 to 10 µm.

Then, in this method, as shown in FIG. 4 (d), the insulating cover layer 14 is formed. For the formation of the insulating cover layer 14, the same method as the method for forming the insulating base layer 13 is used. A preferable method that may be used is the method in which a varnish of a photosensitive synthetic resin is applied on the surface of the insulating base layer 13 so as to cover the conductive pattern 8, photoprocessed, and then cured.
To form the electrode-side openings 15 and the terminal-side opening 16, the insulating cover layer 14 may be formed into a pattern having the electrode-side openings 15 and the terminal-side opening 16; or the electrode-side openings 15 and the terminal-side opening 16 may also be formed, for example, by a discharge processing method, and a laser processing method.

Afterwards, as shown in FIG. 5 (e), the metal foil is trimmed in correspondence with the sections of the metal substrate 12, thereby forming the belt-like circuit board for blood collection 1, and at the same time, openings 3 are made in the metal substrate 12, thereby forming the puncture needle 7 from the metal substrate 12. By trimming in such a manner, the frame portion 17 and the joint portion 18 are simultaneously formed.
For the trimming of the metal foil, for example, discharge processing, laser processing, mechanical punching processing (for example, punching processing), or etching processing is used. In view of easy cleaning after processing, etching processing (wet etching) is preferably used.

In this fashion, the circuit board for blood collection 1 including the plurality of measurement units 2 can be produced in a plural number.
In the obtained circuit board for blood collection 1, as shown in FIG. 5 (f), the chemical agent 19 is applied on the surface of the electrodes 9: that is, glucose oxidase, or glucose dehydrogenase, as an enzyme, and for example, potassium ferricyanide, ferrocene, or benzoquinone as a mediator, alone or in combination is applied. For the application of the chemical agent 19, for example, an appropriate method such as a dipping method, a spray method, or an inkjet method is used.

Depending on the type of the chemical agent 19, it is also possible to, after the plating layer of a different metal is formed on the surface of the electrodes 9 as described above, further form a coating of a different metal in advance, and provide a predetermined potential difference therebetween. To be specific, for example, after a gold plating layer is formed, silver or silver chloride is applied on the surface of the gold plating layer.

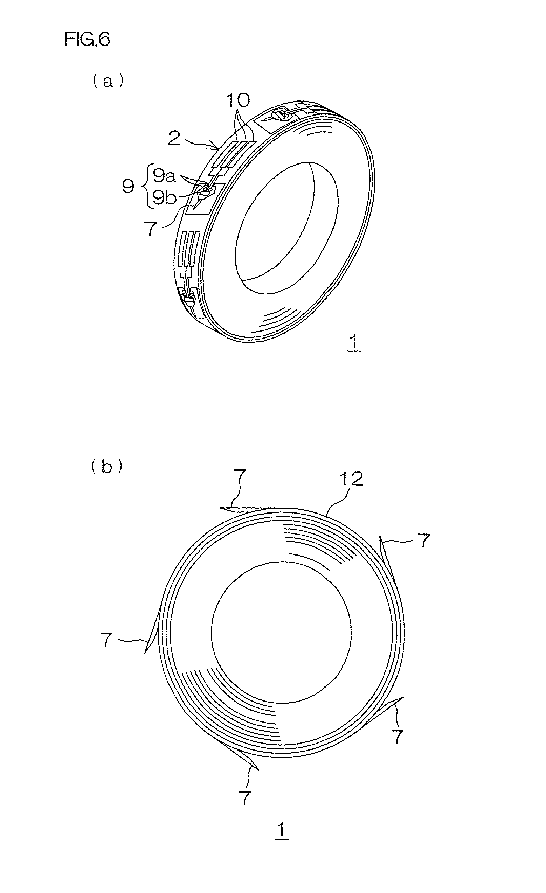

FIG. 6 illustrates an embodiment of a method for using a circuit board for body fluid collection of the present invention: (a) showing a perspective view, and (b) showing a schematic side view along the axial direction. In FIG. 6, only the metal substrate 12 and the conductive pattern 8 are shown for simplicity.

To use the circuit board for blood collection 1, first, the joint portion 18 is cut off, thereby separating the circuit board for blood collection 1 from the frame portion 17, and thereby preparing one strip of circuit board for blood collection 1.
Next, in this method, as shown in FIG. 6 (a), the circuit board for blood collection 1 is wound so that the terminals 10 and the electrodes 9 face toward an outer side in the radial direction.
In this fashion, as shown in FIG. 6 (b), the puncture needle 7 at the outermost layer protrudes so as to tilt toward an outer side in the radial direction from the circumferential direction of the wound circuit board for blood collection 1 due to the elasticity of the metal substrate 12.

The puncture needle 7 disposed at an inner side in the radial direction than the outermost layer is suppressed so as not to protrude to an outer side in the radial direction from the circumferential direction by an adjacent layer at an outer side in the radial direction to the layer in which the puncture needle 7 is disposed.
The length of the wound circuit board for blood collection 1 in the radial direction is, for example, 1 to 10 cm, or preferably 2 to 6 cm. The distal end of the puncture needle 7 of the wound circuit board for blood collection I protrudes toward an outer side in the radial direction by, for example, 1 to 15 mm, or preferably 2 to 8 mm.

The wound circuit board for blood collection 1 is mounted in the blood-sugar level measuring device 21 for a patient to puncture his/her skin of, for example, finger to collect blood, to measure a glucose level in the collected blood.

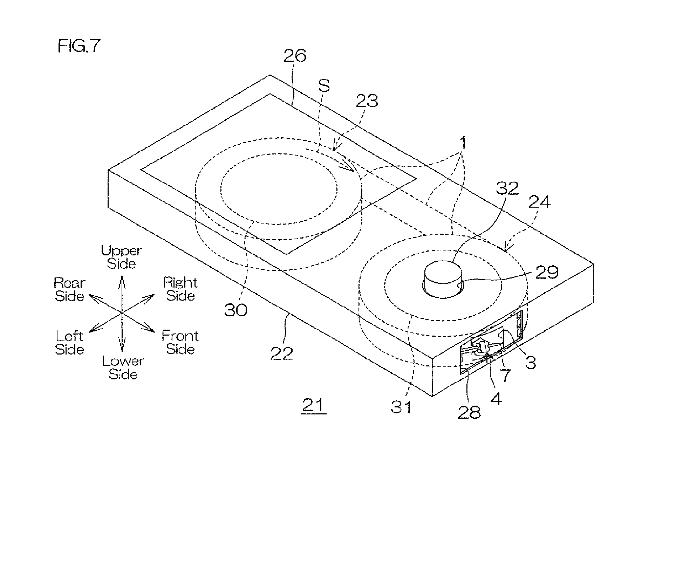

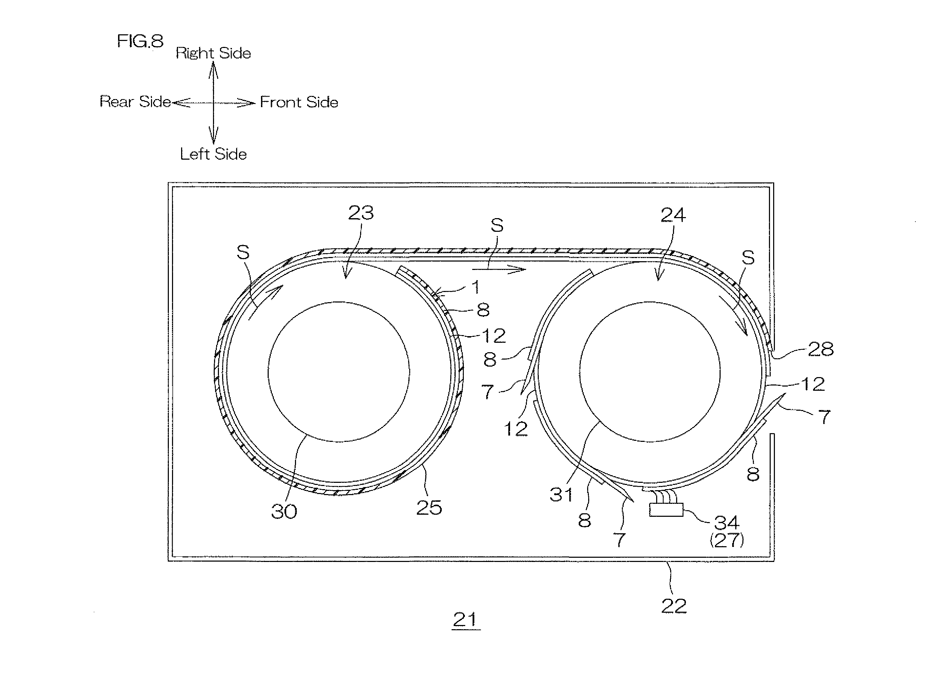

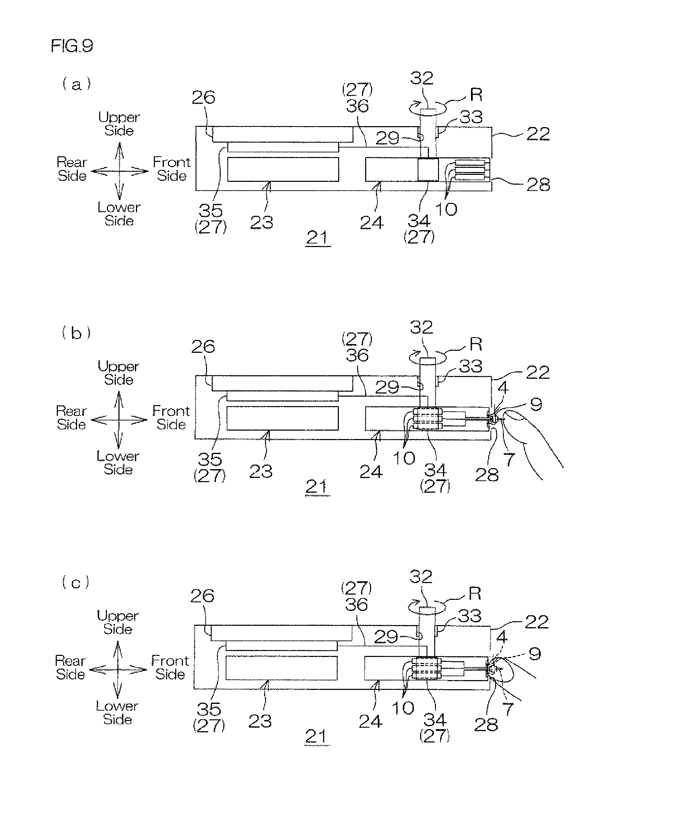

FIG. 7 shows a schematic perspective view of a blood-sugar level measuring device in which the circuit board for blood collection shown in FIG. 6 is mounted as an embodiment of a biosensor of the present invention; FIG. 8 shows a schematic cross-sectional view illustrating inside the blood-sugar level measuring device shown in FIG. 7; and FIG. 9 shows sectional side views for describing a method for using the blood-sugar level measuring device shown in FIG. 7. In FIG. 9, the right side on the plane of the sheet is referred to as "front side", the left side on the plane of the sheet is referred to as "rear side", the upper side on the plane of the sheet is referred to as "upper side", the lower side on the plane of the sheet is referred to as "lower side", the front side on the plane of the sheet is referred to as "left side", and the back side on the plane of the sheet is referred to as "right side", and directions indicated in FIGs. 7 and 8 are in accordance with the directions indicated in FIG. 9. FIG. 8 shows a state where only one layer of the circuit board for blood collection 1 is wound for simplicity, and only the metal substrate 12 and the conductive pattern 8 are shown in the constituent elements of the circuit board for blood collection 1.

Next, with reference to FIGs. 7, 8, and 9, a description of a method for using the circuit board for blood collection 1, and a method for using the blood-sugar level measuring device 21 in which the circuit board for blood collection 1 is mounted is given.
The blood-sugar level measuring device 21 includes, as shown in FIG. 7, a casing 22 as a housing, a feed unit 23, a take-up unit 24, a guide unit 25 (ref: FIG. 8), a display unit 26, and a determination unit 27 (ref: FIG. 9).

The casing 22 is prepared to accommodate the members of the blood-sugar level measuring device 21, and formed into a box. To be specific, the casing 22 accommodates the feed unit 23, the take-up unit 24, the guide unit 25, and the determination unit 27, and on the upper face of the casing 22, the display unit 26 is provided. The casing 22 is formed with a front side opening 28 as an exposure slot and an upper side opening 29.

The length of the casing 22 in up-down direction is, for example, 0.5 to 4 cm, or preferably 1 to 2 cm; the length in the left-right direction is, for example, 2 to 10 cm, or preferably 3 to 7 cm; and the length in the front-rear direction is, for example, 2 to 20 cm, or preferably 5 to 15 cm.
The front side opening 28 is formed on the front wall of the casing 22 so as to extend in the left-right direction to be formed into a generally rectangular shape, and to expose one puncture needle 7 when the take-up unit 24 takes up the circuit board for blood collection 1 in the feed unit 23, as described later.

The upper side opening 29 is formed at a center in the left-right direction of a front side of the upper wall of the casing 22 into a generally circular shape when viewed from top. To be specific, the upper side opening 29 is forked so that a driving shaft 32 to be described later is inserted thereto.
The feed unit 23 includes a feed wheel 30, and is disposed at a rear side of the take-up unit 24 and a lower side of the display unit 26 in the casing 22.

The feed wheel 30 is formed into a generally disk shape, and is supported rotatably in the casing 22.
The circuit board for blood collection 1 is wound around the feed wheel 30 so that the puncture needle 7 is disposed to face toward an upstream-side in feed direction S (ref: FIG. 8), and held thereat.
The take-up unit 24 includes, as shown in FIGs. 7 and 9, a take-up wheel 31, a driving shaft 32, and a bearing unit 33, and is disposed at a front side of the feed unit 23 in the casing 22.

The take-up wheel 31 is formed into a generally disk shape, and the driving shaft 32 is inserted thereto integrally at a center in the radial direction. The front end edge of the take-up wheel 31 is disposed at the rear of the front end face of the casing 22 by, for example, 0.1 to 20 mm, or preferably 2 to 10 mm.
The driving shaft 32 shares the central axis with the take-up wheel 31, extends in up-down direction, and is disposed so as to protrude from the upper wall of the casing 22 through the upper side opening 29.

The bearing unit 33 is provided at a peripheral end of the upper side opening 29 of the casing 22. The bearing unit 33 rotatably supports the driving shaft 32.
The guide unit 25 is provided, as shown in FIG. 8, to extend from the feed unit 23 to the take-up unit 24, and suppresses the puncture needle 7 of the circuit board for blood collection 1 so as not to protrude to an outer side in the radial direction. To be specific, the guide unit 25 is provided along the outer circumference of the feed unit 23, and then extends in the front-rear direction from the right end portion of the feed unit 23 toward the right end portion of the take-up unit 24; and is formed up to the right end edge of the front side opening 28 along the outer circumference of the take-up unit 24.

The guide unit 25 is formed from, for example, plastic and the like, into a flat plate-like shape extending between the lower and upper walls of the casing 22.
The length of the guide unit 25 in up-down direction is made longer than the length of the circuit board for blood collection 1 in the width direction, and is, for example, 5 to 35 mm, or preferably 10 to 20 mm. The thickness of the guide unit 25 is, for example, 0.2 to 5 mm, or preferably 0.5 to 3 mm. The guide unit 25 is disposed, for example, 0.1 to 3 mm, or preferably 0.3 to 1 mm apart from the wound circuit board for blood collection 1 in the radial direction.

The determination unit 27 includes, as shown in FIGs. 8 and 9, a contact portion 34, a CPU 35, and signal wirings 36 that electrically connect the contact portion 34 and the CPU 35.
The contact portion 34 is formed into a brush-like form; is disposed at the left side of the take-up unit 24 and at the outer side in the radial direction of the take-up unit 24 in the casing 22; and is provided slidably with respect to the terminal 10 so as to make contact with the terminal 10 of the measurement unit 2 that performs a measurement when the circuit board for blood collection 1 is in use. That is, the contact portion 34 is provided so as to make contact with the terminal 10 under a state in which the puncture needle 7 of the measurement unit 2 is exposed from the front side opening 28. The contact portion 34 is provided so that a voltage can be applied to the electrodes 9 via the terminals 10, and that a change in a resistance value between the electrodes 9 can be detected upon voltage application when the contact portion 34 is brought into contact with the terminals 10.

The CPU 35 is provided above the feed unit 23 at a lower side of the display unit 26, electrically connected to the contact portion 34 via the signal wirings 36, and electrically connected to the display unit 26. The CPU 35 calculates a glucose level as a blood-sugar level based on the change in the resistance value between the electrodes 9 detected at the contact portion 34 when the circuit board for blood collection 1 performs a measurement.
The display unit 26 is provided, as shown in FIG. 7, at a rear side on the upper wall of the casing 22; includes, for example, LED; and displays the blood-sugar level measured by the CPU 35.

When using the blood-sugar level measuring device 21, first, as shown in FIG. 9 (a), the blood-sugar level measuring device 21 is prepared.
At this time, as shown in FIG. 8, the circuit board for blood collection 1 is wound around the feed wheel 30 in the casing 22; is drawn out along the guide unit 25; and carried over to the take-up wheel 31. The most-upstream-side end portion of the circuit board for blood collection 1 in the puncturing direction is held by the take-up wheel 31. That is, the puncture needle 7 is disposed, facing toward an upstream-side in feed direction S of the circuit board for blood collection 1.

Furthermore, because the guide unit 25 is disposed at the outer circumference of the feed unit 23, the puncture needle 7 of the circuit board for blood collection 1 that is wound around the feed wheel 30 is pressed inward in the radial direction by the guide unit 25, and does not protrude toward an outer side in the radial direction. Similarly, the puncture needle 7 of the circuit board for blood collection 1 that is drawn out from the feed wheel 30 and carried over to the take-up wheel 31 does not protrude as well.
Next, in this method, as shown in FIG. 9 (b), the driving shaft 32 is rotated to winding direction R, thereby exposing the puncture needle 7 from the front side opening 28.

At this time, as shown in FIG. 8, the circuit board for blood collection 1 wound around the feed wheel 30 is fed from the feed unit 23, transported in feed direction S along the guide unit 25, and taken up by the take-up wheel 31 of the take-up unit 24. Then, when the puncture needle 7 is completely exposed from the front side opening 28, the puncture needle 7 is brought away from the guide unit 25, and the puncture needle 7 protrudes toward an outer side of the casing 22 from the front side opening 28 due to elasticity of the metal substrate 11. Then, a patient himself/herself punctures, for example, his/her finger with the protruded puncture needle 7.

At this time, upon puncturing with the puncture needle 7, when the stopper portion 6 makes contact with the skin, further puncturing is restricted. Thus, the puncturing depth of the puncture needle 7 is, for example, 0.5 to 1.5 mm.
Next, in this method, as shown in FIG. 9 (c), the punctured portion is brought closer to and in contact with the electrodes 9.

Then, the surface of the electrodes 9 is brought into contact with the blood collected by the puncturing with the puncture needle 7, and the blood is reacted with the chemical agent 19. At this time, the contact portion 34 is brought into contact with the terminals 10, and at the same time, a voltage is applied to the electrodes 9 from the contact portion 34 via the terminals 10. Then, a change in the resistance value between the electrodes 9 at the time of the voltage application is detected by the contact portion 34, and based on the change in the resistance value detected by the contact portion 34, the CPU 35 calculates a glucose level as a blood-sugar level. Then, the blood-sugar level measured by the CPU 35 is displayed at the display unit 26.

Afterwards, in this method, although not shown, by further rotating the driving shaft 32 in winding direction R, the take-up wheel 31 is rotated, and an unused measurement unit 2 that is disposed upstream of and adjacent to the used measurement unit 2 in feed direction S is exposed from the front side opening 28. Thereafter, the above-described steps shown in FIG. 9 (a) to FIG. 9 (c) are performed a plurality of times, thereby measuring a blood-sugar level a plurality of times.

Then, with the circuit board for blood collection 1, and the blood-sugar level measuring device 21 including the circuit board for blood collection 1, by causing bleeding by puncturing with the puncture needle 7, and allowing the electrodes 9 of the measurement unit 2 to be easily brought into contact with the blood that was caused to bleed, a blood-sugar level can be simply measured by the CPU 35 that is electrically connected to the electrodes 9.
Thus, the circuit board for blood collection 1 and the blood-sugar-level measuring device 21 are capable of simply measuring a blood-sugar level with a simple structure.

Furthermore, with the circuit board for blood collection 1, based on the plurality of measurement units 2 provided in one strip of circuit board for blood collection 1, multiple measurements of a blood-sugar level can be achieved.
Furthermore, by winding the circuit board for blood collection 1 and disposing the plurality of measurement units 2 along the circumferential direction, and by rotating the circuit board for blood collection 1 in the circumferential direction after using one measurement unit 2, the used measurement unit 2 can be changed to an unused measurement unit 2 that is upstream of and adjacent to the used measurement unit 2 in the rotational direction. Therefore, for every measurement in the multiple measurements, the measurement unit 2 can be changed easily.

Furthermore, with the above-described method for producing the circuit board for blood collection 1, a plurality of measurement units 2 can be formed in a manner such that the measurement units 2 are arranged in line along the longitudinal direction of the belt-like metal substrate 12. Therefore, yields of the circuit board for blood collection 1 can be improved, and an improvement in production efficiency allows reduction in costs.
Furthermore, with the above-described method for using the circuit board for blood collection 1, by winding the circuit board for blood collection 1, the puncture needle 7 can be protruded to tilt toward an outer side in the radial direction from the circumferential direction. Therefore, the puncture needle 7 can be protruded with simple steps. As a result, the puncture needle 7 is disposed at an outer side in the radial direction of the wound circuit board for blood collection 1, and therefore reliable puncturing and measurement by the measurement unit 2 can be achieved.

Furthermore, with the above-described blood-sugar level measuring device 21, as the take-up unit 24 is rotated and takes up the circuit board for blood collection 1, the measurement unit 2 moves from the feed unit 23 to the take-up unit 24. At this time, the guide unit 25 provided to extend from the feed unit 23 to the take-up unit 24 suppresses the protrusion of the puncture needle 7.
Then, when the measurement unit 2 reaches the front side opening 28, the puncture needle 7 is brought away from the guide unit 25, thereby protruding toward an outer side in the radial direction, and being exposed from the front side opening 28. Thus, reliable puncturing and measurement by the measurement unit 2 can be achieved.

Furthermore, with the above-described blood-sugar level measuring device 21, the puncture needle 7 is disposed to face toward an upstream-side in feed direction S of the circuit board for blood collection 1, and therefore both of the direction of the puncture needle 7 and the direction of the pressing force of pressing by the guide unit 25 on the puncture needle 7 face toward an upstream-side in feed direction S. Therefore, the puncture needle 7 can fend off the pressing force from the guide unit 25 by getting away along the guide unit 25 toward an inner side in the radial direction of the circuit board for blood collection 1 that is wound. Thus, the circuit board for blood collection 1 can be transported smoothly from the feed unit 23 to the take-up unit 24 without damage to the circuit board for blood collection 1 or the puncture needle 7.

In the description above, description is given by using the circuit board for blood collection 1, and the blood-sugar level measuring device 21 including the circuit board for blood collection 1 as examples of the circuit board for body fluid collection, and the biosensor including the circuit board for body fluid collection of the present invention. That is, description is given using blood as the body fluid collected by puncturing with the puncture needle of the circuit board for body fluid collection.
However, the body fluid is not particularly limited as long as it is a liquid in a living body, and examples thereof include extracellular fluid and intracellular fluid. Examples of the extracellular fluid include, other than blood mentioned above, a blood plasma; an intercellular fluid; a lymph fluid; moistures in dense connective tissue, bone, and cartilage; and a transcellular fluid. A measurement on a specific component of the above-described body fluid can be performed with the circuit board for body fluid collection and the biosensor including the circuit board for body fluid collection.

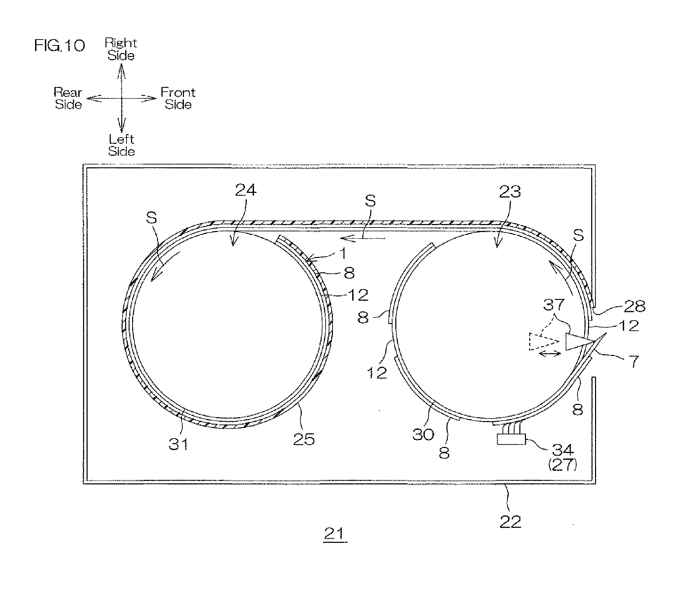

Furthermore, in the description above, the puncture needle 7 is protruded so as to tilt toward an outer side in the radial direction from the circumferential direction by winding the circuit board for blood collection 1. However, when the puncture needle 7 does not sufficiently protrude for some reason, for example, the thickness of the metal substrate 12 is small, as shown in FIG. 10, a pushing guide 37 may be provided at an inner side in the radial direction of the wound circuit board for blood collection 1 so that the puncture needle 7 can be protruded from an inner side in the radial direction to an outer side in the radial direction by pushing it out with the pushing guide 37.

The circuit board for blood collection 1 can also be moved from the front side toward the rear side along feed direction S, by disposing the feed wheel 30 at a front side and disposing the take-up wheel 31 to a rear side.
In FIG. 10, the feed wheel 30 is disposed at a front side, and the take-up wheel 31 is disposed at a rear side, i.e., the component corresponding to the take-up unit 24 in FIG. 8 is replaced with the feed unit 23, and the component corresponding to the feed unit 23 in FIG. 8 is replaced with the take-up unit 24. In FIG. 10, an embodiment in which only one layer of the circuit board for blood collection 1 is wound is shown. The members that are the same as those in FIG. 8 are designated by the same reference numerals used in FIG. 8, and descriptions thereof are omitted.

### EXAMPLES

### EXAMPLE 1

### (Production of Circuit Board for Blood Collection)

First, an elongated sheet metal foil made of SUS 304, in which metal substrates were defined, and having a thickness of 50 µm was prepared (ref: FIG. 4 (a)).
Then, on the metal substrate, a varnish of a photosensitive polyimide resin precursor (photosensitive polyamic acid resin) was applied, and dried by heating to form a coating. The coating was then exposed to light, and developed to be formed into a pattern. Thereafter, the coating was heated in a nitrogen atmosphere to 400°C, to form an insulating base layer having a thickness of 10 µm in the abovementioned pattern (ref: FIG. 4 (b)).

Then, on the insulating base layer, metal thin films formed of a chromium thin film and a copper thin film were formed sequentially by sputtering. Subsequently, a dry film resist was laminated on the surface of the metal thin film, exposed to light, and developed to form a plating resist in a pattern. Then, a plating layer formed of copper was formed on the surface of the metal thin film exposed from the plating resist using the metal thin film as a seed film by electrolytic copper plating, thereby forming a conductive pattern including electrodes, terminals, and wirings (ref: FIG. 4 (c)). Afterwards, the plating resist and the portion of the metal thin film where the plating resist was formed was removed by etching.

The thickness of the conductive pattern was 12 µm, the diameter of the two electrodes (9a) was 0.3 mm, and the length of the long side of the one electrode (9b) was 1.0 mm, and the short side thereof was 0.6 mm. The length in the width direction of the end portion in the puncturing direction of the wirings was 100 µm. The space in the width direction between the adjacent wirings was, at an upstream side in the puncturing direction, 3 mm, and at a downstream-side in the puncturing direction, 0.3 mm.
Afterwards, a varnish of a photosensitive polyimide resin precursor (photosensitive polyamic acid resin) was applied on the insulating base layer so as to cover the conductive pattern, and dried by heating to form a coating. The coating was then exposed to light, and developed to be formed into a pattern. Afterwards, the coating was heated in a nitrogen atmosphere to 400°C to form an insulating cover layer having a thickness of 5 µm (ref: FIG. 4(d)). The insulating cover layer was formed such that by forming the electrode-side openings and terminal-side opening, the electrodes and the terminals were exposed but the wirings were covered.

Thereafter, an electrolytic nickel plating layer (thickness 0.5 µm), and an electrolytic gold plating layer (thickness 2.5 µm) were sequentially formed on the electrodes and the terminals.
Then, a dry film resist was laminated on the surfaces of the metal foil (upper face and/or lower face), exposed to light, and developed to form an etching resist in a pattern. Then, the metal foil exposed from the etching resist was etched by wet etching using ferric chloride as an etchant to be trimmed, thereby forming a belt-like circuit board for blood collection. At the same time, openings were formed in the metal substrate, thereby forming puncture needles from the metal substrate (ref: FIG. 5 (e)). By such trimming of the metal foil, the frame portion and the joint portion were formed simultaneously.

The length from the distal end of the puncture needle to the one electrode (9b)(the electrode nearest from the distal end) was 1.8 mm, the angle of the distal end of the puncture needle was 20°, the spaced apart-length from the downstream-side end edge of the stopper portion in the puncturing direction to the distal end of the puncture needle was 1.4 mm.
In this fashion, the circuit board for blood collection in which the puncturing direction of the puncture needles of the measurement units is all the same direction was obtained. The length of the circuit board for blood collection in the width direction was 5 mm, and the length in the puncturing (longitudinal) direction was 20 cm.

Afterwards, in the obtained circuit board for blood collection, a chemical agent containing glucose oxidase and a potassium ferricyanide solution was applied on the electrode in respective measurement units by inkjet (ref: FIG. 5 (f)).

### (Production of Blood-Sugar Level Measuring Device)

The obtained circuit board for blood collection was removed from the frame portion by cutting off the joint portion, and the circuit board for blood collection was wound around the feed wheel so that the electrodes and the terminals face toward an outer side in the radial direction (ref: FIG. 6 (a)).

The length of the wound circuit board for blood collection in the radial direction was 4 cm. The distal end of the puncture needle in the wound circuit board for blood collection protruded toward an outer side in the radial direction by 5 mm.
The feed wheel around which the circuit board for blood collection was wound was mounted in the casing in which the take-up wheel, the driving shaft, the guide unit, and the display unit were provided (ref: FIGs. 7 and 8).
To mount the circuit board for blood collection, first, the feed wheel around which the circuit board for blood collection was wound was disposed in the feed unit; and then the circuit board for blood collection wound around the feed wheel was drawn out along the guide unit, and carried over to the take-up wheel, allowing the most-upstream-side end portion of the circuit board for blood collection in the puncturing direction to be held by the take-up wheel.

The length of the casing in the up-down direction was 1.5 cm; the length in the left-right direction was 5 cm; and the length in the front-rear direction was 10 cm.
The front end edge of the take-up wheel was disposed at a rear of the front end face of the casing by 5 mm.
The guide unit was made of plastic; and had a length in the up-down direction of 10 mm, and a thickness of 0.5 mm. The guide unit is formed, with a space of 0.5 mm provided between the guide unit and the wound circuit board for blood collection in the radial direction.

### (Blood-Sugar Level Measurement with Blood-Sugar Level Measuring Device)

First, the above-described blood-sugar level measuring device was prepared (ref: FIG. 9(a)).
Then, by rotating the driving shaft in the winding direction, the puncture needle was exposed from the front side opening, and a patient himself/herself punctured his/her finger with the puncture needle (ref: FIG. 9 (b)).
Then, the electrodes were brought closer to the punctured portion, thereby bringing the electrodes into contact with blood (ref: FIG. 9 (c)).

Then, glucose was oxidized by the blood, and ferricyanide ions reacted. At the same time, a voltage was applied from the contact portion to the electrodes via the terminals. Then, a change in the resistance value between the electrodes at the time of the voltage application was detected by the contact portion, and the CPU calculated a glucose level as a blood-sugar level based on the change in the resistance value. Then, the blood-sugar level measured by the CPU was displayed at the display unit.
Afterwards, in this method, by further rotating the driving shaft in the winding direction, and thereby rotating the take-up wheel, an unused measurement unit that is disposed upstream of and adjacent to the used measurement unit in the feed direction was exposed from the front side opening. Thereafter, the above-described steps were performed, thus measuring a blood-sugar level a plurality of times.

While the illustrative embodiments of the present invention are provided in the above description, such is for illustrative purpose only and it is not to be construed as limiting the scope of the present invention. Modification and variation of the present invention that will be obvious to those skilled in the art is to be covered by the following claims.

### Industrial Applicability

The circuit board for body fluid collection, the method for producing the same, the method for using the same, and the biosensor including the circuit board for body fluid collection of the present invention are useful, for example, for application in testing devices for collecting a liquid in a living body such as blood.

## Claims

1. A circuit board for body fluid collection comprising:
a belt-like supporting substrate, and
a plurality of measurement units defined in the supporting substrate along a longitudinal direction of the supporting substrate,
wherein the plurality of measurement units each include
a puncture needle formed from the supporting substrate along the longitudinal direction of the supporting substrate by forming an opening in the supporting substrate,
an insulating base layer formed on the supporting substrate, and
a conductive layer formed on the insulating base layer and including an electrode for making contact with a body fluid collected by puncturing with the puncture needle.

2. The circuit board for body fluid collection according to Claim 1,
wherein when the circuit board for body fluid collection is wound, the puncture needle protrudes so as to tilt toward an outer side in the radial direction from a circumferential direction.

3. A biosensor comprising:
a circuit board for body fluid collection including a belt-like supporting substrate, and a plurality of measurement units defined in the supporting substrate along a longitudinal direction of the supporting substrate, wherein the plurality of measurement units each include a puncture needle formed from the supporting substrate along the longitudinal direction of the supporting substrate by forming an opening in the supporting substrate, an insulating base layer formed on the supporting substrate, and a conductive layer formed on the insulating base layer and including an electrode for making contact with a body fluid collected by puncturing with the puncture needle;
a housing in which an exposure slot for exposing the puncture needle is formed,
a feed unit in which the circuit board for body fluid collection is wound, and that feeds the circuit board for body fluid collection,
a take-up unit that takes up the circuit board for body fluid collection that is fed from the feed unit; and
a guide unit that is provided to extend from the feed unit to the take-up unit, and suppresses the puncture needle of the circuit board for body fluid collection not to protrude toward an outer side in the radial direction.

4. The biosensor according to Claim 3, wherein
the puncture needle is disposed to face toward an upstream-side in a feed direction of the circuit board for body fluid collection.

5. A method for producing a circuit board for body fluid collection, the method comprising the steps of:
preparing a belt-like supporting substrate in which a plurality of measurement units are defined in a longitudinal direction with a space provided therebetween,
forming an insulating base layer on the plurality of measurement units,
forming a conductive layer including an electrode for making contact with a body fluid on the insulating base layer, and
forming a puncture needle from the supporting substrate along the longitudinal direction of the supporting substrate by forming an opening in the supporting substrate.

6. A method for using a circuit board for body fluid collection, the method comprising the steps of:
winding a circuit board for body fluid collection produced by a method for producing a circuit board for body fluid collection, the method for producing a circuit board for body fluid collection comprising the steps of:
preparing a belt-like supporting substrate in which a plurality of measurement units are defined in a longitudinal direction with a space provided therebetween,
forming an insulating base layer on the measurement units,
forming a conductive layer including an electrode for making contact with a body fluid on the insulating base layer, and
forming a puncture needle from the supporting substrate along the longitudinal direction of the supporting substrate by forming an opening in the supporting substrate.
